# EUROPEAN PATENT APPLICATION

(11) **EP 4 356 878 A1**
(43) Date of publication of application: **24.04.2024**
(21) Application number: 23460004.7
(22) Date of filing: 20.02.2023
(51) Int. Cl.: A61F 2/915

(54) **SELF-EXPANDING STENT**

(30) Priority: 17.10.2022 PL 44253922
(71) Applicant: Stentpoint P sp.z o.o., 91-134 Lódz (PL)
(72) Inventor: Mizera, Ireneusz, 98 - 220 Zdunska Wola (PL)
(74) Representative: Dziubinska, Joanna

(57) **Abstract**

Self-expanding stent characterized in that has a closed-cell structure at its ends and it has an open-cell structure in the middle section; in the closed-cell section all stent meshes of the stent are connected to adjacent ones, while in the open-cell section only some meshes are connected to adjacent ones; the open-cell section is created by connecting the V-shaped elements to make a circle in the shape of a crown, and then the individual circles forming the length are connected by every fourth corner to the corner of the preceding circle, unlike the closed-cell section, where the circles forming the length are connected to all previous corners; in addition, the device at both ends in the closed-cell section expands to increase its diameter with respect to the central open-cell section.

## Description

The subject of the invention is a new and innovative self-expanding stent intended for the surgeries of blood vessel diseases with particular emphasis on type A aortic dissections.

Various types of devices used during cardiac and endovascular surgeries are known.

Self-expanding and nitinol stents are known.

According to the invention, the self-expanding stent is preferably made of nitinol by laser cutting method. Nitinol is an alloy of nickel and titanium and its characteristic ability is shape memory. This allows the product to be firmly packed into a narrow insertion system into the patient's body allowing it to reach the desired release site through the blood vessels. After being released by body heat, the stent recovers its original shape.

The essence of the patent is that the stent is a closed-cell structure at its ends and it has an open-cell structure in the middle section; in the closed-cell section all stent meshes of the stent are connected to adjacent ones, while in the open-cell section only some meshes are connected to adjacent ones; the open-cell section is created by connecting the V-shaped elements to make a circle in the shape of a crown, and then the individual circles forming the length are connected by every fourth corner to the corner of the preceding circle, unlike the closed-cell section, where the circles forming the length are connected to all previous corners; in addition, the device at both ends in the closed-cell section expands to increase its diameter with respect to the central open-cell section.

The stent is a closed-cell structure at its ends and it has an open-cell structure in the middle section. This structure ensures good fixation of the stent in the blood vessel with closed-cell elements at the ends, while the central part is flexible, which is ensured by the open-cell elements.

An aortic dissection is a condition in which a tear occurs in the innermost layer of the aorta. Blood rushes though the tear, creating a false flow channel between the layers of the aorta. This leads to restriction of blood flow in the true channel and flow disturbances in the branches from the aorta. An aortic dissection is one of the most serious complications of untreated or poorly controlled hypertension.

According to the invention, the self-expanding stent is designed to expand the true flow channel to its correct size, which at the same time is to lead to complete narrowing of the false channel. The expansion takes place due to the self-expanding property of the device under the influence of body temperature, i.e. by increasing its diameter from a few millimetres to tens of millimetres planned for a specific piece, because of the use of the metal with shape memory. By placing and releasing the stent in the true channel, the stent increases its diameter and expands the channel and at the same time the false channel is closed.

A type A aortic dissection is a condition that begins in the ascending aorta, which runs from the heart up towards the neck, where the cranial vessels depart from it and the aorta begins to descend down towards the abdomen, forming a segment of the descending aorta.

In the known state of the art, the only method of treating type A dissections is a very extensive and burdensome surgery consisting in opening the patient's chest in order to resect the damaged fragment of the ascending aorta and replace it with an artificial prosthesis to which the cranial vessels are sutured. In this way, the influx of blood to the dissection is eliminated.

A type A dissection is formed in the first section of the aorta, i.e. in the ascending aorta, the second section is the aortic arch, from which the vessels supplying the head with blood depart.

According to the invention, the self-expanding stent, as a result of the insertion of the stent in the area of the cranial vessels, allows for avoiding long-term surgeries under full anaesthesia by shortening the time of the procedure to make it safer. The self-expanding stent according to the invention solves this problem by the use of the endovascular method, which is often performed even under local anaesthesia. The solution according to the invention can be inserted into the patient's body by opening the iliac artery a few millimetres, which is done in the groin. A 6-7 mm thick insertion system where the stent is packed is placed through this entrance. The system is long enough to slide through the iliac artery into the main aorta to the ascending part reaching the heart. There, the stent is released and opened, thus closing the blood inflow to the dissection, expanding the main flow, eliminating the false one, and then closing the outflow from the false channel in the descending aorta, by closing the false channel completely. The entire dissection is supplied with one element, the implantation of which takes several minutes, and the entire operation, including patient preparation and finishing activities, can take about an hour.

The solutions known from the state of the art relate to self-expanding metal stents with shape memory and balloon expansion. Neither of these solutions is applicable to type A dissections. The stent in the invention overcomes these disadvantages. The stent is self-expanding, as the aorta grows over time, and due to this property it can respond to these aortic changes and continue to expand to its maximum size. In contrast, a balloon-expandable stent remains in the size to which it has been expanded.

The self-expanding stent according to the invention must be of the appropriate diameter to capture the aorta and must be flexible to evenly compress the aorta to close the false lumen.

The solution according to the invention preserves the structure of the aorta and the natural blood flow. The diameter and length of the stent are known in the art.

The self-expanding stent according to the invention must be matched in diameter to the diameter of the patient's aorta in order to fulfil its task. This stent has three types of connections of adjacent stent meshes. The open-cell section has two types of mesh connections (1) and (2). The first one (1) is the connection of every fourth mesh with the adjacent circle, while the second one (2) is a place without a connection, therefore it is called an open-cell connection. The closed-cell section has one type of connection (3). This type (3) is the connection of the stent meshes at all corners and this connection forms closed rhombuses.

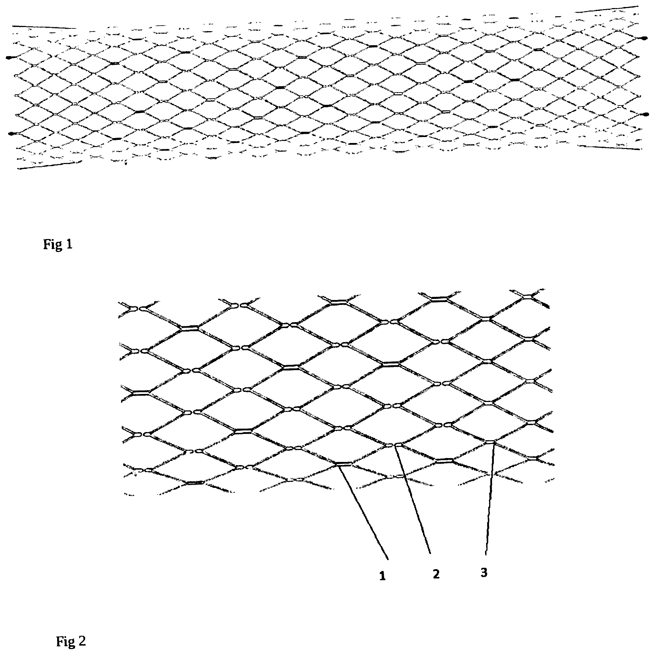

The solution according to the invention is shown in the accompanying illustrations, figs 1-2, where fig. 1 shows a general view of the stent, fig. 2 shows an enlarged close-up of a fragment of the stent.

The solution according to the invention is shown in non-limiting examples of claims.

### Example No. 1:

The self-expanding stent according to the invention preferably has a length of 195-200 mm. The middle section of the device has an open-cell structure and preferably has a length of about 125 - 130 mm. The outer parts of the solution have a closed-cell structure and preferably have a length of 35 - 40 mm. The width of the whole device varies between 35 - 45 mm. The closed-cell section slightly expands towards its ends. The diameter of the stent at both ends in the closed-cell section expands by about 5 mm, compared to the central open-cell section. The meshes in the open-cell section are connected by connecting (1) - every fourth mesh with the adjacent circle. In the open-cell section there are also contact points (2), which are places without connections. On the other hand, the closed-cell sections has one type of connection (3). This type (3) is the connection of the stent meshes at all corners and this connection forms closed rhombuses.

## Claims

1. Self-expanding stent consisting of a flexible stent, **characterized in that** has a closed-cell structure at its ends and it has an open-cell structure in the middle section; in the closed-cell section all stent meshes of the stent are connected to adjacent ones, while in the open-cell section only some meshes are connected to adjacent ones; the open-cell section is created by connecting the V-shaped elements to make a circle in the shape of a crown, and then the individual circles forming the length are connected by every fourth corner to the corner of the preceding circle, unlike the closed-cell section, where the circles forming the length are connected to all previous corners; in addition, the device at both ends in the closed-cell section expands to increase its diameter with respect to the central open-cell section.

## Amended claims

### Amended claims in accordance with Rule 137(2) EPC.

1. Self-expanding stent consisting of a flexible stent, **characterized in that** has a closed-cell structure at its ends and it has an open-cell structure in the middle section; in the closed-cell section all stent meshes of the stent are connected to adjacent ones, while in the open-cell section only some meshes are connected to adjacent ones; the open-cell section is created by connecting the V-shaped elements to make a circle in the shape of a crown, and then the individual circles forming the length are connected (1) by every fourth corner to the corner of the preceding circle, unlike the closed-cell section, where the circles forming the length are connected (3) to all previous corners; in addition, the device at both ends in the closed-cell section expands to increase its diameter with respect to the central open-cell section.
